(19) Europäisches Patentamt
European Patent Office
Office européen des brevets

(11) EP 0 736 059 B1

(12) EUROPÄISCHE PATENTSCHRIFT

(45) Veröffentlichungstag und Bekanntmachung des Hinweises auf die Patenterteilung:
**30.07.1997 Patentblatt 1997/31**

(21) Anmeldenummer: **95902117.1**

(22) Anmeldetag: **02.12.1994**

(51) Int Cl.[6]: **C08G 85/00**, A61K 49/00

(86) Internationale Anmeldenummer:
**PCT/EP94/04016**

(87) Internationale Veröffentlichungsnummer:
**WO 95/17451 (29.06.1995 Gazette 1995/27)**

(54) **METALLKOMPLEXE VON DENDRIMEREN MAKROMOLEKÜLEN, DIESE ENTHALTENDE DIAGNOSTISCHE MITTEL SOWIE VERFAHREN ZUR HERSTELLUNG DER KOMPLEXE UND MITTEL**

METAL COMPLEXES OF DENDRIMERIC MOLECULES, DIAGNOSTIC AGENTS CONTAINING THEM AND PROCESS FOR PRODUCING THE COMPLEXES AND AGENTS

COMPLEXES METALLIQUES DE MACROMOLECULES DENDRIMERES, MOYENS DE DIAGNOSTIC CONTENANT CES COMPLEXES ET PROCEDE POUR LA PREPARATION DES COMPLEXES ET DES MOYENS DE DIAGNOSTIC

(84) Benannte Vertragsstaaten:
**AT BE CH DE DK ES FR GB GR IE IT LI LU MC NL PT SE**

(30) Priorität: **22.12.1993 DE 4344460**

(43) Veröffentlichungstag der Anmeldung:
**09.10.1996 Patentblatt 1996/41**

(73) Patentinhaber: **SCHERING AKTIENGESELLSCHAFT**
**13353 Berlin (DE)**

(72) Erfinder:
- **SCHMITT-WILLICH, Heribert**
**D-12161 Berlin (DE)**
- **PLATZEK, Johannes**
**D-14195 Berlin (DE)**
- **MÜHLER, Andreas**
**D-10319 Berlin (DE)**
- **FRENZEL, Thomas**
**D-12247 Berlin (DE)**

(56) Entgegenhaltungen:
**WO-A-91/05762**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft den in den Patentansprüchen gekennzeichneten Gegenstand, das heißt neue Metallkomplexe von dendrimeren Makromolekülen, diese Verbindungen enthaltende Mittel, die Verwendung der Komplexe in der Diagnostik sowie Verfahren zur Herstellung dieser Komplexe und Mittel.

Magnevist® (Gd-DTPA/dimeglumin) ist das erste registrierte Kontrastmittel für die Kernspintomographie (MRI = Magnetic Resonance Imaging). Es ist besonders gut für die Diagnose pathologischer Bereiche (z. B. Entzündungen, Tumore) geeignet. Die Verbindung wird nach intravenöser Injektion über die Nieren eliminiert; eine extrarenale Ausscheidung wird praktisch nicht beobachtet.

Ein Nachteil von Magnevist® ist, daß es sich nach intravenöser Applikation gleichmäßig zwischen dem vasalen und dem interstitiellen Raum verteilt. Somit ist eine Abgrenzung der Gefäße gegenüber dem umliegenden interstitiellen Raum nicht möglich.

Für Perfusionsuntersuchungen ist ein Kontrastmittel notwendig, welches sich ausschließlich im vasalen Raum (Gefäßraum) verteilt. Ein solches "blood-pool-agent" soll es ermöglichen, mit Hilfe der Kernspintomographie gut durchblutetes von schlecht durchblutetem Gewebe abzugrenzen und somit eine Ischämie zu diagnostizieren.

Weitere Anwendungsgebiete sind die Gefäßdarstellung mit Hilfe der Kernspintomographie (die sogenannte MR-Angiographie) und die bildliche Darstellung von Permeabilitätsstörungen von Blutgefäßen (wie z.B. in bösartigen Tumoren).

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen. In der Angiographie wird zur Zeit vor allem die Röntgen-Diagnostik mit Hilfe von iodhaltigen Kontrastmitteln angewandt. Diese Untersuchungen sind mit verschiedenen Nachteilen behaftet:
Sie sind mit dem Risiko der Strahlenbelastung, sowie mit Unannehmlichkeiten und Belastungen verbunden, die vor allem daher kommen, daß die iodhaltigen Kontrastmittel, verglichen mit NMR-Kontrastmitteln, in sehr viel höherer Konzentration angewandt werden müssen und nicht im Vasalraum verbleiben.

Es besteht daher ein Bedarf an NMR-Kontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität beim MRI) auszeichnen.

Der Ansatz, zumindest einen Teil dieser Probleme durch Verwendung von Komplexbildnern, die an Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen 0 088 695 und 0 150 884 beschrieben sind, für eine zufriedenstellende Bildgebung nicht ausreichend.

Makromoleküle sind generell als Kontrastmittel für die Angiographie geeignet. Albumin-Gd-DTPA (Radiology 1987; 162:205) z.B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

In der EP 0 233 619 werden blood-pool-agents auf der Basis von Polylysin-Gd-DTPA beschrieben. Diese Verbindungen sind jedoch mit dem Nachteil behaftet, daß nur eine unbefriedigende Ausscheidung der Komplexe und damit der in den Komplexen enthaltenen Schwermetallionen erfolgt.

Eine Verbesserung bezüglich der Ausscheidbarkeit brachten die in der EP 0 430 863 beschriebenen Kaskadenpolymer-Komplexe. Jedoch erfolgt auch hier in einem vertretbaren Zeitraum keine vollständige Ausscheidung, so daß die Gefahr der Freisetzung des Metalls aus dem Komplex besteht.

Auch makromolekulare Kontrastmittel auf der Basis von Kohlenhydraten, z.B. Dextran, sind beschrieben worden (Europäische Patentanmeldung 0 326 226). Der Nachteil dieser Verbindungen liegt darin, daß sie in der Regel nur 4,6 % des signalverstärkenden paramagnetischen Kations tragen.

Aufgabe der vorliegenden Erfindung war es daher, neue diagnostische Mittel vor allem zur Erkennung von Gefäßerkrankungen zu finden, die die genannten Nachteile nicht besitzen, d.h. Metallkomplexe zu finden die nach intravenöser Applikation im Untersuchungszeitraum ein geringes Diffusionsvermögen durch die Gefäßwandungen zeigen und dennoch quantitativ ausgeschieden werden.

Diese Aufgabe wird durch die vorliegende Erfindung gelöst.

Es wurde gefunden, daß dendrimere Polymer-Komplexe der Formel I

$$A\text{-}(X)_b \qquad (I)$$

worin

A für einen stickstoffhaltigen Kern der Basismultiplizität b steht, wobei

b die Summe der freien Valenzen des stickstoffhaltigen Kerns bedeutet und für Ziffern 1 bis 8 steht und
X für einen aus

$$\sum_{k=0}^{n-1} 2^k$$

Reproduktionseinheiten S und $2^n$ bildgebenden Resten Y zusammengesetzten Rest steht
worin

n die Anzahl der Generationen bestimmt und für die Ziffern 1, 2, 3 oder 4 steht,
S für einen Rest der Formel II steht,

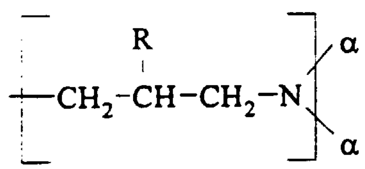

(II)

worin

R für ein Wasserstoffatom oder eine Methylgruppe steht und die Positionen
α für 0 ≤ k ≤ n-1 durch weitere Reproduktionseinheiten S, und für die n-te Generation durch bildgebende Reste Y der Formel III oder IV besetzt sind,

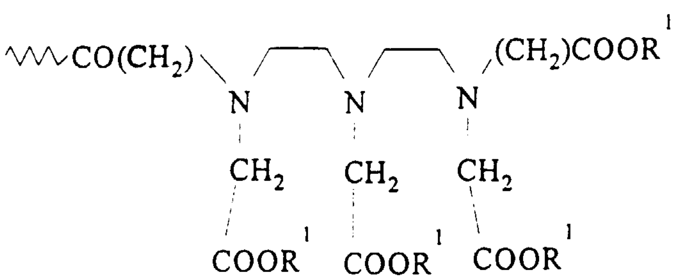

(III)

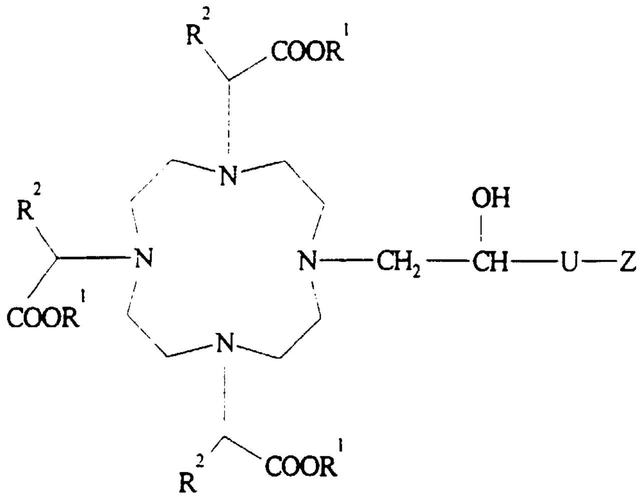

(IV)

worin

$R^1$ unabhängig voneinander Wasserstoff oder ein Metallionenequivalent der Elemente der Ordnungszahlen 21-29, 39, 42-44 oder 57-83,

$R^2$ für ein Wasserstoffatom, einen Methyl- oder einen Ethylrest, der gegebenenfalls mit 1- 2 Hydroxylgruppen substituiert ist,

U für eine gegebenenfalls Imino-, Phenylen-, Phenylenoxy-, Phenylenimino-, Amid-, Hydrazid-, Carbonyl-, Estergruppen, Sauerstoff-, Schwefel- und/oder Stickstoff-Atom(e) enthaltende, gegebenenfalls durch Hydroxy-, Mercapto-,Oxo-, Thioxo-, Carboxy-, Carboxyalkyl-, Ester- und/oder Aminogruppe(n) substituierte geradkettige, verzweigte, gesättigte oder ungesättigte $C_1$-$C_{20}$ Alkylengruppe und

Z für eine -CO-, -NH-CO- oder -NHCS-Gruppe steht,

wobei die Komplexe mindestens 8 - 64 Metallionen der genannten Elemente enthalten und freie Carbonsäuregruppen gewünschtenfalls als Salz einer anorganischen oder organischen Base oder Aminosäure vorliegen, überraschenderweise hervorragend als NMR-Diagnostika zur Kontrastierung des vasalen Raums geeignet sind, ohne die genannten Nachteile aufzuweisen.

Die dendrimeren Polymerkomplexe der vorliegenden Erfindung setzen sich also zusammen aus einem 1 bis 8 Stickstoffatome enthaltenden Kern, dessen freie Valenzen in der ersten Generation jeweils mit einer 3-Aminopropyl- oder einer 3-Amino-2-methyl-propyl- Gruppe - den sogenannten Reproduktionseinheiten S - abgesättigt sind.

Den einfachsten dendrimeren Kern stellt ein Stickstoffatom dar, dessen drei freie Valenzen (Basismultiplizität b = 3) in der ersten Generation mit drei Reproduktionseinheiten besetzt sind (bzw. dessen drei Wasserstoffatome des zugrundeliegenden Ammoniaks durch die drei Reproduktionseinheiten S substituiert sind). Jede dieser drei Reproduktionseinheiten enthält ein terminales Stickstoffatom, dessen (beiden) freie Valenzen entweder

a) vollständig durch die bildgebenden Reste der Formel III oder IV abgesättigt sind oder

b) durch jeweils eine weitere Reproduktionseinheit S besetzt sind.

Diese zweite Generation besteht im Falle des vorgenannten Beispiels eines Stickstoffkerns aus 6 (3 x 2) Reproduktionseinheiten mit ebenso vielen terminalen Stickstoffatomen. Die insgesamt 12 (6 x 2) freien Valenzen der 2-ten Generation können nun entweder

a) durch die bildgebenden Reste der Formel III oder IV abgesättigt sein oder

b) wiederholt durch identische Reproduktionseinheiten S besetzt werden, so daß eine dritte Generation entsteht. Dieser 3-ten Generation kann sich gewünschtenfalls eine vierte anschließen.

Erfindungsgemäß geeignet sind dendrimere Polymer-Komplexe, die aus maximal 4 mindestens jedoch aus einer Generation bestehen. Die letzte (bzw. n-te) Generation weist dabei $2^n/2$ multipliziert mit der Basismultiplizität b terminale Stickstoffatome mit $2^n$ Positionen $\alpha$ auf, die vollständig mit bildgebenden Resten der Formel III oder IV besetzt sind. Dabei sind sowohl die Reproduktionseinheiten als auch die bildgebenden Reste innerhalb eines Moleküls identisch.

Die Anzahl der insgesamt im Molekül enthaltenen Reproduktionseinheiten wird durch die Anzahl der Generationen festgelegt und berechnet sich nach der Formel

$$b \cdot \sum_{k=0}^{n-1} 2^k ,$$

worin b für die Basismultiziplität, n für die Anzahl der Generationen und k für eine Laufziffer steht die von 0 bis n-1 läuft. So enthält also z.B. ein aus drei Generationen (n = 3) bestehendes Polymer insgesamt

$$b \cdot \sum_{k=0}^{3-1} 2^k \quad [= b \cdot (2^0 + 2^1 + 2^2)] = b \times 7$$

identische Reproduktionseinheiten.

Für den vorgenannten Fall des Stickstoffkerns mit der Basismultiplizität b = 3 ergeben sich insgesamt also 21 (3 x 7) Reproduktionseinheiten S. Zusätzlich enthält das Polymer b x $2^n$ bildgebende Reste, im konkreten Fall also 24 (3 x $2^3$).

X steht somit also für einen Zweig des dendrimeren polymeren Komplexes, der sich aus der Summe der Reproduktionseinheiten und den dazugehörigen bildgebenden Resten Y zusammensetzt.

Als Kaskadenkerne A kommen prinzipiell beliebige stickstoffhaltige Kerne, mit maximal 8 freien Valenzen an den Stickstoffatomen, infrage. Neben dem genannten Stickstoffkern seien beispielhaft genannt eine

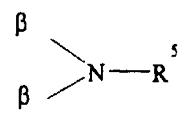

oder eine β-N-$(R^5)_2$ Gruppe. Besonders geeignet ist jedoch ein Rest der allgemeinen Formel V, VI, VII, oder VIII,

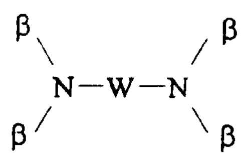

(V)

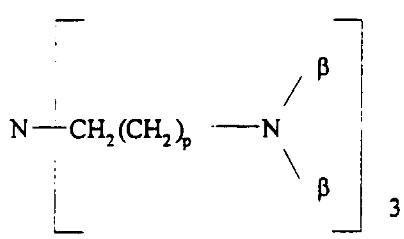

(VI)

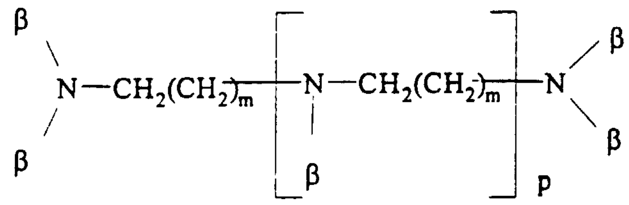

(VII)

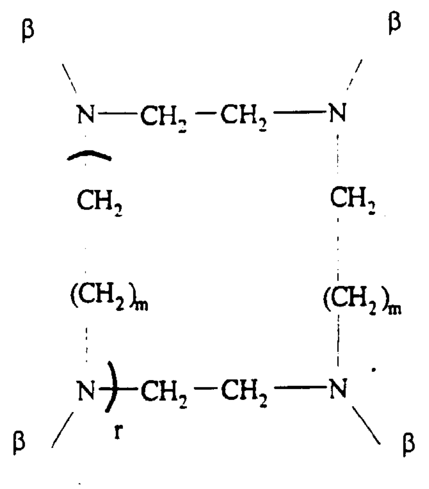

(VIII)

worin

- $R^5$ für einen gegebenenfalls mit 1-4 OH-Gruppen substituierten Alkyl-, Aryl- oder Aralkylrest mit bis zu 12 C-Atomen steht,
- β die Bindungsstelle zum Rest X markiert, wobei die Anzahl der mit der Basismultiplizität b gleichzusetzen ist,
- W für einen geradkettigen oder verzweigten Alkylen-, Arylen- oder Aralkylenrest mit bis zu 12 C-Atomen steht, der gegebenenfalls durch 1-4 Sauerstoffatome unterbrochen und/oder durch 1-4 Hydroxygruppen substituiert ist,
- p für die Ziffern 1 bis 4,
- m unabhängig voneinander für die Ziffern 1 oder 2 und
- r für die Ziffern 1 bis 5 steht.

Als Beispiele für die dem Kaskadenkern A zugrundeliegenden Amine $A(H)_b$ seien Ammoniak, Trisaminoethylamin, 1,4,7,10-Tetraazacyclododecan (Cyclen), 1,4,7,10,13,16-Hexaazacyclooctadecan, 1,3-Diaminopropan, 1,4-Diaminobutan, 1,5-Diaminopentan, 1,12-Diamino-4,9-dioxadodccan, 1,4,8,11-Tetraazaundecan, 1,5,8,12-Tetraazadodecan, 1,5,9,13-Tetraazatridecan, Diethylentriamin oder Triethylentetramin genannt.

Bevorzugt sind darunter Ammoniak, Tris(2-aminoethyl)amin, Diethylentriamin oder Cyclen, insbesondere jedoch 1,4-Diaminobutan.

Die Basismultiplizität β ergibt sich aus der Anzahl der Stickstoff-Wasserstoffbindungen des dem jeweiligen Kern entsprechenden Amins $A(H)_b$. So hat Ammoniak die Basismultiplizität 3, das Tris(2-aminoethyl)amin die Basismultiplizität 6, das Diethylentriamin die Basismultiplizität 5 und das 1,4,7,10-Tetraazacyclododecan die Basismultiplizität 4.

Die Herstellung der erfindungsgemäßen dendrimeren polymeren Komplexe der allgemeinen Formel I erfolgt, indem man ein dendrimeres Polymer mit terminalen (endständigen) Aminogruppen der Formel IX,

$$A\text{-}(X')_b \qquad (IX)$$

worin

A und b die in Anspruch 1 angegebenen Bedeutung haben und
X' für X steht, wobei jedoch im Unterschied zu X für die n-te Generation die Positionen $\alpha$ nicht durch die bildgebenden Reste Y, sondern durch Wasserstoffatome besetzt sind, mit einer reaktiven Vorstufe des Komplexbildners oder bildgebenden Komplexes in einer Acylierungs- oder Additionsreaktion umsetzt und anschließend - falls es sich bei der reaktiven Vorstufe um einen Komplexbildner handelt - mit Metallsalzen bzw. Metalloxiden der genannten Metalle zu dem gewünschten polymerverknüpften Komplex umsetzt.

Die als Ausgangsverbindung eingesetzten Aminogruppen-haltigen dendrimeren Polymere der Formel IX werden wie in der WO 93/14147 offenbart, hergestellt.

Sollen dendrimere polymere Komplexe von bildgebenden Resten der Formel III erhalten werden, so findet als reaktive Vorstufe in der Regel das Monoanhydrid der Diethylentriaminpentaessigsäure (J. Pharm. Sci., **68** (1979) 194) Verwendung. Dieses wird analog zu den in der DE 42 32 925 offenbarten Verfahren zu den entsprechenden Amid-verknüpften Verbindungen umgesetzt wird.
Diese Umsetzung wird in flüssiger Phase in Gegenwart einer Base durchgeführt. Geeignete Reaktionsmedien sind beispielsweise Wasser, polare Lösungsmittel wie Tetrahydrofuran, Dioxan, Acetonitril, N-Methylpyrrolidon, Formamid, Dimethylformamid, Dimethylacetamid oder Gemische derselben. Die Umsetzung wird vorzugsweise bei pH 8-10, d.h. unter Zusatz von Basen wie z.B. Natrium- oder Kaliumhydroxid oder Triethylamin, bei Temperaturen von 0 - 50 °C, vorzugsweise bei Raumtemperatur, durchgeführt. Zur vollständigen Umsetzung arbeitet man vorzugsweise mit einem 2 - 3-fachen Überschuß Anhydrid. Anschließend werden die so erhaltenen Komplexbildner, in an sich bekannter Weise, mit Metallsalzen oder Metalloxiden zu den erfindungsgemäßen Komplexen umgesetzt. Sollen hingegen dendrimere polymere Komplexe von bildgebenden Resten der Formel IV erhalten werden, so wird als reaktive Vorstufe in der Regel eine Verbindung der allgemeinen Formel X

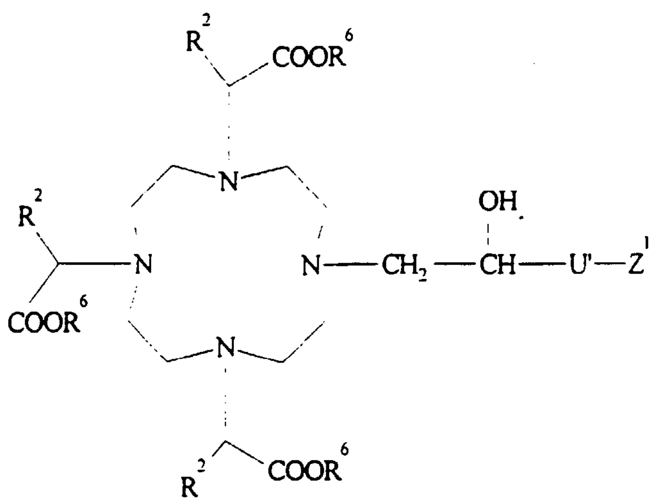

(X)

worin

$R^2$ die angegebene Bedeutung hat und
U' für U oder für eine Vorstufe von U steht,
$R^6$ für eine Säureschutzgruppe steht und/oder ein Metallionenequivalent bedeutet und
$Z^1$ für eine Isocyanat-, eine Isothiocyanat-, eine aktivierte Säuregruppe oder ein Lactonrest steht,

eingesetzt.

Für den Fall, daß $R^6$ für eine Säureschutzgruppe steht, wird diese im Anschluß an die Acylierung- oder Additionsreaktion in an sich bekannter Weise abgespalten und die so erhaltenen Verbindungen mit Metalloxiden oder Metallsalzen zu den erfindungsgemäßen Komplexen umgesetzt.

Als Beispiele für eine aktivierte Säuregruppe seien beispielhaft Anhydrid, p-Nitrophenylester, N-Hydroxysuccimidester, Säurechlorid und eine durch ein Carbodiimid-Derivat in situ aktivierte Carbonsäure genannt.

Als Säureschutzgruppen $R^6$ kommen niedere Alkyl-, Aryl- und Aralkylgruppen, wie beispielsweise Methyl-, Ethyl-, Propyl-, n-Butyl-, t-Butyl-, Phenyl-, Benzyl-, Diphenylmethyl-, Triphenylmethyl-, bis(p-Nitophenyl)-methyl- oder Trialkylsilylgruppen in Frage.

Die Abspaltung der Säureschutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 - 50 °C, saure Verseifung mit Mineralsäuren oder im Fall von z.B. tert.-Butylestern mit Hilfe von Trifluoressigsäure.

Die Acylierung erfolgt in flüssiger Phase in Gegenwart von anorganischen und/oder organischen Basen. Geeignete Reaktionsmedien sind z.B. Tetrahydrofuran, Dioxan, Acetonitril, Formamid, DMF, DMSO, Dimethylacetamid, Wasser oder Gemische derselben.

Additionsreaktionen von Isothiocyanaten mit den gewünschten Aminen des dendrimeren Polymeren werden in der Regel in polaren Lösungsmitteln, wie beispielsweise Wasser, Alkoholen wie z.B. Methanol, Ethanol oder Isopropanol, Tetrahydrofuran, Dioxan, Acetonitril, N-Methylpyrrolidon, Formamid, Dimethylformamid, Dimethylacetamid oder deren Gemischen, durchgeführt.

Analoge Additionsreaktionen von Isocyanaten werden vorzugsweise in wasserfreier flüssiger Phase nach literaturbekannten Verfahren durchgeführt (Houben-Weyl, Methoden der Organischen Chemie, Georg-Thieme-Verlag, Stuttgart, New York, Bd. E4 (1983) S. 768-784).

Die Herstellung der Metallkomplexe aus den Komplexbildnern erfolgt in der Weise, wie sie in den Patentschriften EP 0 071 564, EP 0 130 934 und DE 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21-29, 39, 42-44 oder 57-83 in Wasser und/oder einem niederen Alkohol (wie Methanol, Ethanol, Isopropanol und/oder N,N-Dimethylformamid) löst oder suspendiert und mit der Lösung oder Suspension der äquivalenten Menge des Komplexbildners umsetzt.

Zur Erreichung eines physiologischen pH-Werts können abschließend acide Wasserstoffatome von Säure-Gruppen durch Kationen anorganischer und/oder organischer Basen oder Aminosäuren substituiert werden.

Als Basen kommen in Frage anorganische Basen (z. B. Hydroxide, Carbonate oder Bicarbonate) von z. B. Natrium, Kalium oder Lithium und/oder organische Basen wie unter anderem primäre, sekundäre und tertiäre Amine wie z.B. Ethanolamin, Morpholin, Glucamin, N-Methyl- und N,N-Dimethylglucamin, sowie basische Aminosäuren wie z. B. Lysin, Arginin und Ornithin.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z. B. niederen Alkoholen (z.B. Methanol, Ethanol, Isopropanol), niederen Ketonen (z.B. Aceton), polaren Ethern (z.B. Tetrahydrofuran, Dioxan, 1,2-Dimethoxyethan) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Ein weiterer Gegenstand der Erfindung sind Mittel, welche mindestens eine der erfindungsgemäßen Verbindungen enthalten.

Die Erfindung betrifft weiterhin ein Verfahren zur Herstellung dieser Mittel, welches dadurch gekennzeichnet ist, daß der in Wasser gelöste dendrimere Polymer-Komplex mit den in der Galenik üblichen Zusätzen und Stabilisatoren in eine für die enterale bzw. parenterale Applikation geeignete Form gebracht wird, so daß der Komplex in einer Konzentration von 0,01 bis 1,0 Mol/l vorzugsweise in einer Konzentration von 0,1 bis 0,5 Mol/l vorliegt. Die resultierenden Mittel werden anschließend gewünschtenfalls sterilisiert. Sie werden in Abhängigkeit der diagnostischen Fragestellung in der Regel in einer Dosis von 0,01 - 0,3 mmol/kg Körpergewicht appliziert.

Geeignete Zusätze sind beispielsweise physiologisch unbedenkliche Puffer (wie z. B. Tromethamin oder Diethylentriaminpentaessigsäure), geringe Zusätze des jeweiligen polymeren, dendrimeren Komplexbildners, gegebenenfalls in Form eines physiologisch verträglichen Salzes, wie z.B. des Kalziumsalzes sowie Elektrolyte wie z. B. Natriumchlorid und/oder gewünschtenfalls Antioxidantien wie z. B. Ascorbinsäure.

Prinzipiell ist es auch möglich, die erfindungsgemäßen diagnostischen Mittel auch ohne Isolierung der polymeren Komplexverbindung herzustellen. In jedem Fall muß besondere Sorgfalt darauf verwendet werden, die Komplexierung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Als letzte Sicherheit bleibt eine Reinigung des isolierten Komplexsalzes.

Weitere Gegenstände der Erfindung sind durch die Ansprüche gekennzeichnet.

Die erfindungsgemäßen Substanzen erfüllen die vielfältigen Voraussetzungen, die an ein "blood-pool-agent" in der NMR-Diagnostik zu stellen sind. Die Verbindungen und aus ihnen hergestellte Mittel zeichnen sich aus durch:

- eine günstige Ausscheidungskinetik,
- eine gute Verträglichkeit,
- eine hohe Wirksamkeit, die notwendig ist, um den Körper mit möglichst geringen Mengen an Fremdstoffen zu belasten,
- geringe Osmolalität.

Insbesondere verbleiben die erfindungsgemäßen Verbindungen (bzw. Mittel) während des Untersuchungszeitraum ausschließlich im vasalen Raum, so daß eine Unschärfe im NMR-Bild, durch in den interstitiellen Raum diffundierendes Kontrastmittel, nicht beobachtet wird. Weiterhin weisen die erfindungsgemäßen Mittel nicht nur eine hohe Stabilität in vitro auf, sondern auch eine überraschend hohe Stabilität in vivo, so daß eine Freigabe oder ein Austausch der in den Komplexen gebundenen - an sich giftigen - Ionen innerhalb der Zeit, in der die neuen Kontrastmittel vollständig wieder ausgeschieden werden, nicht erfolgt.

Das nachfolgende Beispiel dient der näheren Erläuterung der Erfindungsgegenstandes, ohne ihn auf dieses beschränken zu wollen.

Die nachfolgend benutzte verkürzte Schreibweise "Diaminobutyl-Dendrimer-$(NH_2)_{32}$" steht für ein Dendrimer, das aus einem Diaminobutyl-Kern A und 60 Reproduktionseinheiten ($-CH_2-CH_2-CH_2-N <$) mit 32 terminalen $NH_2$-Gruppen aufgebaut ist.

**Beispiel 1**

a) *[10]-Carboxy-3,6,9-tris(carboxymethyl)-3,6,9-triazadecanoyl]-Derivat des Polyaminodendrimers Diaminobulyl-Dendrimer-$(NH_2)_{32}$*

3,51 g (1 mmol) des in Beispiel VIII der WO 93/14147 beschriebenen 32-Amins werden in 300 ml Wasser gelöst. Innerhalb 2 Stunden wrden anschließend 36,02 g (96 mmol) N3-(2,6-Dioxomorpholinoethyl)-$N^6$-(ethoxycarbonylmethyl)-3,6-diazaoctandisäure (Beispiel 13a der EP 0 331 616) portionsweise in fester Form zugegeben, wobei der pH-Wert durch Zugabe von 1 N NaOH bei 8,5 gehalten wird. Nach Beendigung der Anhydrid-Zugabe wird die Lösung noch 2 Stunden bei pH 11 gerührt, dann stellt man mit Amberlite® IR 120 ($H^+$-Form) auf pH 5 und saugt vom Ionenaustauscher ab. Die Lösung wird ultrafiltriert (AMICON® YM 05-Membran) und das Retentat anschließend gefriergetrocknet.
Ausbeute: 15,6 g
$H_2O$-Gehalt (Karl-Fischer): 9,3 %
100 mg des wasserfreien Komplexbildners komplexieren
(Indikator: Xylenolorange) 29,6 mg $Gd^{3+}$(Besetzungsgrad mit DTPA > 91 %)

b) *Gd-Komplex des [10-Carboxy-3,6,9-tris(carboxymethyl)-3,6,9-triazadecanoyl]-Derivats des Polyaminodendrimers Diaminobutyl-Dendrimer-$(NH_2)_{32}$*

10,0 g des im vorstehenden Beispiel beschriebenen Komplexbildners werden in 300 ml $H_2O$ gelöst und mit 3,41 g $Gd_2O_3$ (=2,96 g Gd) versetzt, 30 Minuten bei 80° C gerührt, nach Abkühlen auf pH 7 eingestellt, membranfiltriert und gefriergetrocknet.
Ausbeute: 12,5 leicht gelbliches, flockiges Lyophilisat
$H_2O$-Gehalt (Karl-Fischer): 8,2 %
Gd-Bestimmung (AAS): 22,9 %

| Analyse (berechnet auf wasserfreie Substanz): | | | | | |
|---|---|---|---|---|---|
| ber.: | C 37,08% | H 4,80% | Gd 24,58% | N 10,81% | Na 0,22% |
| gef.: | C 37,83 % | H 5,19% | Gd 23,10% | N 10,97% | Na 0,51% |

**Patentansprüche**

1. Dendrimere Polymer-Komplexe der Formel I

$$A\text{-}(X)_b \quad (I)$$

worin

A für einen stickstoffhaltigen Kern der Basismultiplizität b steht, wobei
b die Summe der freien Valenzen des stickstoffhaltigen Kerns bedeutet und für Ziffern 1 bis 8 steht und
X für einen aus

$$\sum_{k=0}^{n-1} 2^k$$

Reproduktionseinheiten S und $2^n$ bildgebenden Resten Y zusammengesetzten Rest steht
worin

n die Anzahl der Generationen bestimmt und für die Ziffern 1, 2, 3 oder 4 steht,
S für einen Rest der Formel II steht,

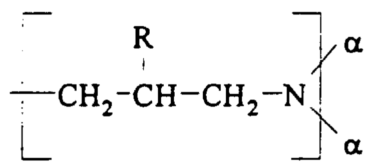

(II)

worin

R für ein Wasserstoffatom oder eine Methylgruppe steht und die Positionen
α für $0 \leq k \leq n-1$ durch weitere Reproduktionseinheiten S, und für die n-te Generation durch bildgebende Reste Y der Formel III oder IV besetzt sind,

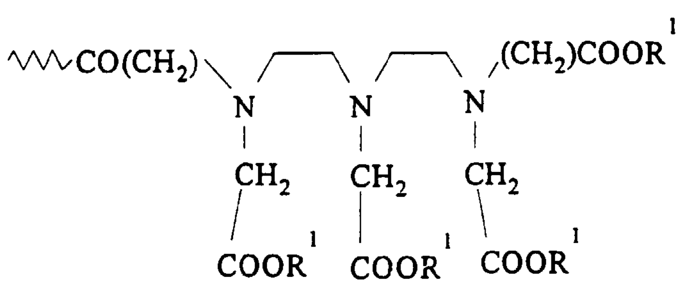

(III)

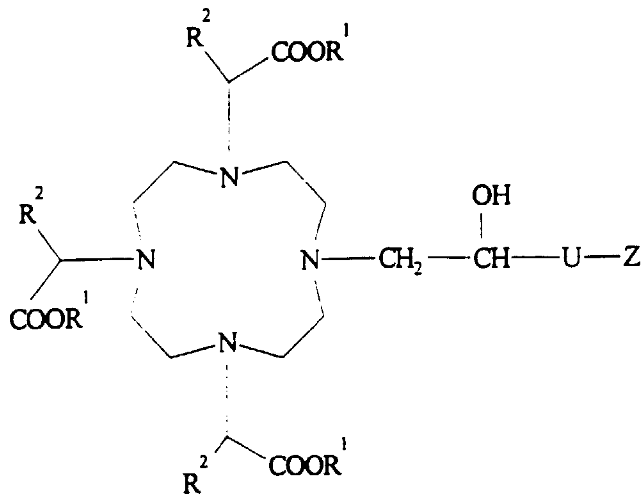

(IV)

worin
$R^1$ unabhängig voneinander Wasserstoff oder ein Metallionenequivalent der Elemente der Ordnungszahlen 21-29, 39, 42-44 oder 57-83,
$R^2$ für ein Wasserstoffatom, einen Methyl- oder einen Ethylrest, der gegebenenfalls mit 1 - 2 Hydroxylgruppen substituiert ist,
U für eine gegebenenfalls Imino-, Phenylen-, Phenylenoxy-, Phenylenimino-, Amid-, Hydrazid-, Carbonyl-, Estergruppen, Sauerstoff-, Schwefel- und/oder Stickstoff-Atom(e) enthaltende, gegebenenfalls durch Hydroxy-, Mercapto-,Oxo-, Thioxo-, Carboxy-, Carboxyalkyl-, Ester- und/oder Aminogruppe(n) substituierte geradkettige, verzweigte, gesättigte oder ungesättigte $C_1$-$C_{20}$ Alkylengruppe und
Z für eine -CO-, -NH-CO- oder -NHCS-Gruppe steht,

wobei die Komplexe mindestens 8 - 64 Metallionen der genannten Elemente enthalten und freie Carbonsäuregruppen gewünschtenfalls als Salz einer anorganischen oder organischen Base oder Aminosäure vorliegen.

**2.** Dendrimere Polymerkomplexe gemäß Anspruch 1, dadurch gekennzeichnet, daß der Kern A für ein Stickstoffatom, einen Rest

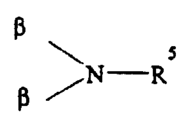

,

β-N-$(R^5)_2$ oder einen Rest der allgemeinen Formel V, VI, VII, oder VIII steht,

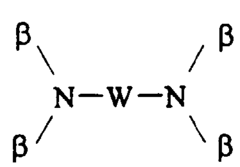

(V)

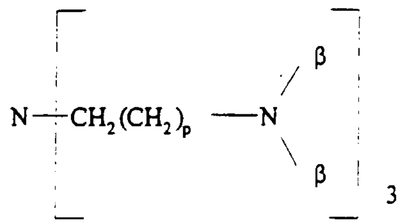

(VI)

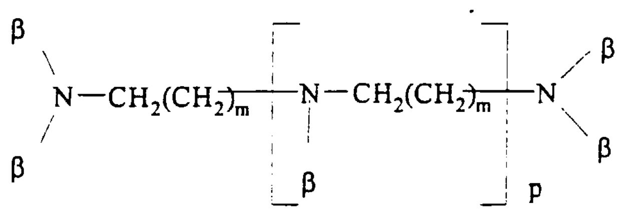

(VII)

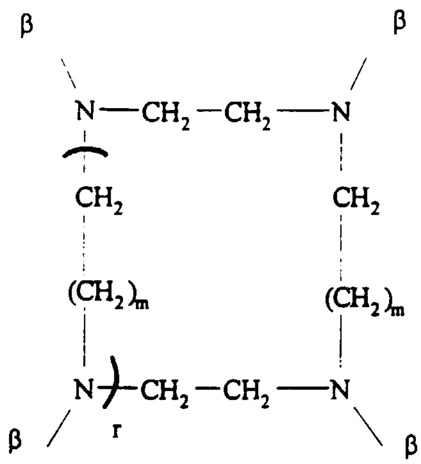

(VIII)

worin

- $R^5$ für einen gegebenenfalls mit 1-4 OH-Gruppen substituierten Alkyl-, Aryl- oder Aralkylrest mit bis zu 12 C-Atomen steht,
- β die Bindungsstelle zum Rest X markiert, wobei die Anzahl der β mit der Basismultiplizität b gleichzusetzen ist,
- W für einen geradkettigen oder verzweigten Alkylen-, Arylen- oder Aralkylenrest mit bis zu 12 C-Atomen steht, der gegebenenfalls durch 1-4 Sauerstoffatome unterbrochen und/oder durch 1-4 Hydroxygruppen substituiert ist,
- p für die Ziffern 1 bis 4,
- m unabhängig voneinander für die Ziffern 1 oder 2 und
- r für die Ziffern 1 bis 5 steht.

**3.** Dendrimere Polymerkomplexe gemäß Anspruch 1 und 2, dadurch gekennzeichnet, daß der Kern A für eine Gruppe > $N-(CH_2)_4-N$ < steht.

**4.** Diagnostische Mittel enthaltend mindestens einen dendrimeren Polymer-Komplex nach Anspruch 1-3 in einem physiologisch verträglichen Medium, gegebenenfalls mit den in der Galenik üblichen Zusätzen.

**5.** Verwendung eines dendrimeren Polymer-Komplexes nach Anspruch 1 zur Herstellung von Mitteln für die NMR- oder Röntgen-Diagnostik.

**6.** Verfahren zur Herstellung eines dendrimeren Polymer-Komplexes nach Anspruch 1-3, dadurch gekennzeichnet, daß dendrimere Polymere der Formel IX

$$A\text{-}(X')_b \qquad \text{(IX)}$$

worin

A und b die angegebenen Bedeutungen haben und
X' für X steht, wobei jedoch im Unterschied zu X für die n-te Generation die Positionen α nicht durch die bildgebenden Reste Y, sondern durch Wasserstoffatome besetzt sind, mit einer reaktiven Vorstufe des Komplexbildners oder bildgebenden Komplexes in einer Acylierungs- oder Additionsreaktion umsetzt und anschließend, falls es sich bei der Reaktiven Vorstufe um einen Komplexbildner handelt, mit Metallsalzen oder Metalloxiden eines Metalls der genannten Ordnungzahlen zu den gewünschten Komplexen umsetzt.

**7.** Verfahren nach Anspruch 6 enthaltend als reaktive Vorstufe einen eine aktivierte Carbonsäure-, Isocyanat- oder Isothiocyanatgruppe enthaltenden Komplexbildner oder Komplex.

**Claims**

**1.** Dendrimeric polymer complexes of formula I

$$A\text{-}(X)_b \qquad \text{(I)}$$

wherein

A represents a nitrogen-containing nucleus of base multiplicity b, with
b denoting the total number of free valencies of the nitrogen-containing nucleus and representing a number from 1 to 8, and
X representing a radical composed of

$$\sum_{k=0}^{n-1} 2^k$$

reproduction units S and $2^n$ image-producing radicals Y
wherein

n defines the number of generations and represents the number 1, 2, 3 or 4, and
S represents a radical of formula II

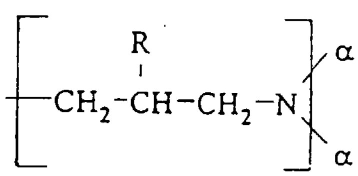

(II)

wherein

R represents a hydrogen atom or a methyl group, and the

α entities are occupied in the case where $0 \leq k \leq n-1$ by further reproduction units S and in the case of the nth generation by image-producing radicals Y of formula III or IV

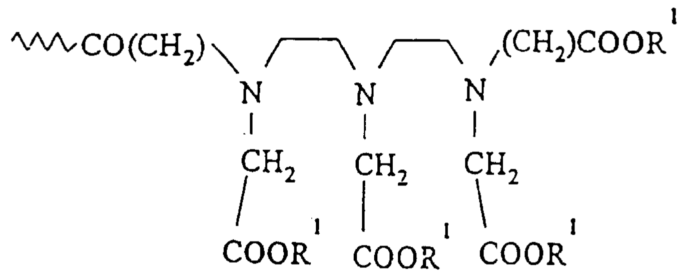

(III)

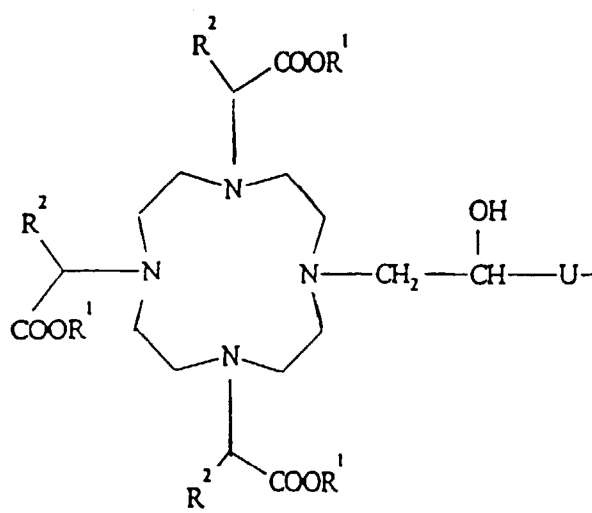

(IV)

wherein

each $R^1$ represents, independently of the others, hydrogen or a metal ion equivalent of an element having an atomic number of from 21 to 29, 39, from 42 to 44 or from 57 to 83,

$R^2$ represents a hydrogen atom, or a methyl or ethyl radical that is unsubstituted or substituted by 1 or 2 hydroxy groups,

U represents a straight-chained or branched, saturated or unsaturated $C_1$-$C_{20}$alkylene group which optionally contains imino, phenylene, phenyleneoxy, phenyleneimino, amide, hydrazide, carbonyl, ester groups, oxygen, sulphur and/or nitrogen atom(s) and which is unsubstituted or substituted by (a) hydroxy, mercapto, oxo, thioxo, carboxy, carboxyalkyl, ester and/or amino group(s), and

Z represents a -CO-, -NH-CO- or -NHCS- group,

the complexes containing from at least 8 to 64 metal ions of the mentioned elements and, if desired, free carboxylic acid groups being present in the form of a salt of an inorganic or organic base or amino acid.

**2.** Dendrimeric polymer complexes according to claim 1, characterised in that the nucleus A represents a nitrogen atom, a

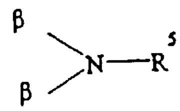

or a β-N-$(R^5)_2$ radical, or a radical of the general formula V, VI, VII or VIII

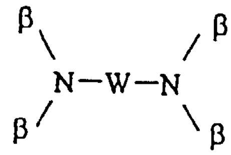

(V)

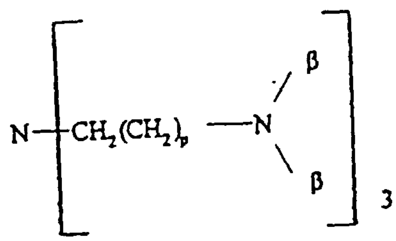

(VI)

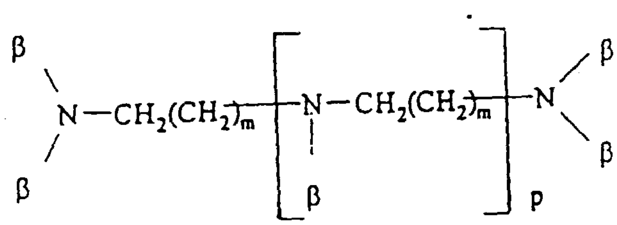

(VII)

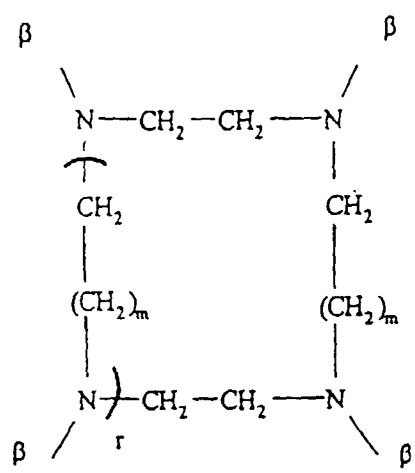

(VIII)

wherein

$R^5$ represents an alkyl, aryl or aralkyl radical having up to 12 carbon atoms that is unsubstituted or substituted by from 1 to 4 OH groups,
β marks the position of the bond to the radical X, the number of β's being the same as the base multiplicity b,
W represents a straight-chained or branched alkylene, arylene or aralkylene radical having up to 12 carbon atoms that is optionally interrupted by from 1 to 4 oxygen atoms and/or substituted by from 1 to 4 hydroxy groups,
p represents a number from 1 to 4,
each mrepresents, independently of the other, the number 1 or 2, and
r represents a number from 1 to 5.

**3.** Dendrimeric polymer complexes according to claims 1 and 2, characterised in that the nucleus A represents a group $>N-(CH_2)_4-N<$.

**4.** Diagnostic compositions comprising at least one dendrimeric polymer complex according to claims 1 to 3 in a physiologically tolerable medium, optionally together with the additives customary in galenical pharmacy.

**5.** Use of a dendrimeric polymer complex according to claim 1 for the preparation of compositions for NMR or X-ray diagnostics.

**6.** Process for the preparation of a dendrimeric polymer complex according to claims 1 to 3, characterised in that dendrimeric polymers of formula IX

$$A\text{-}(X')_b \qquad \text{(IX)}$$

wherein

A and b have the meanings given and
X' represents X but, unlike X, in the case of the nth generation the α entities are occupied not by image-producing radicals Y but by hydrogen atoms,
are reacted with a reactive precursor of the complexing agent or image-producing complex in an acylation or addition reaction and then, where the reactive precursor is a complexing agent, the products are reacted with metal salts or metal oxides of a metal having one of the mentioned atomic numbers to form the desired complexes.

**7.** Process according to claim 6 comprising as the reactive precursor a complex or complexing agent comprising an activated carboxylic acid group, isocyanate group or isothiocyanate group.

**Revendications**

**1.** Complexes polymères dendrimères de formule I

$$A\text{-}(X)_b \qquad \text{(I)}$$

dans laquelle

A représente un noyau azoté à multiplicité de bases b,
b étant la somme des valences libres du noyau azoté et vaut 1 à 8 et
X représente un radical composé de

$$\sum_{k=0}^{n-1} 2^k$$

unités de reproduction S et de $2^n$ éléments inducteurs d'image Y,
où

n détermine le nombre de générations et vaut 1, 2, 3 ou 4,
S représente un radical de formule II

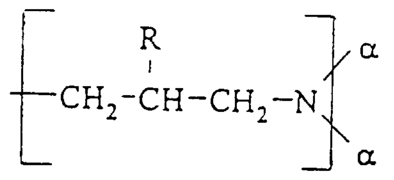

(II)

où

R représente un atome d'hydrogène ou un groupe méthyle et les positions
α sont occupées pour $0 \leq k \leq n-1$ en plus par autres unités de reproduction S et pour la n-ième génération, par des éléments inducteurs d'image Y de formule III ou IV,

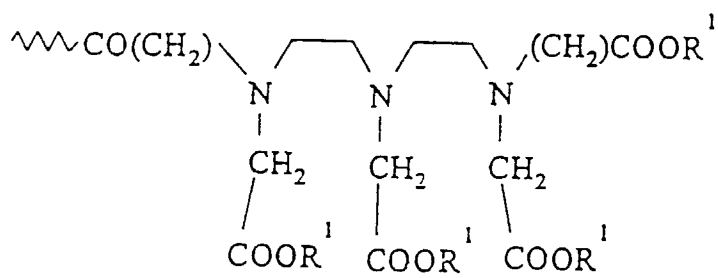

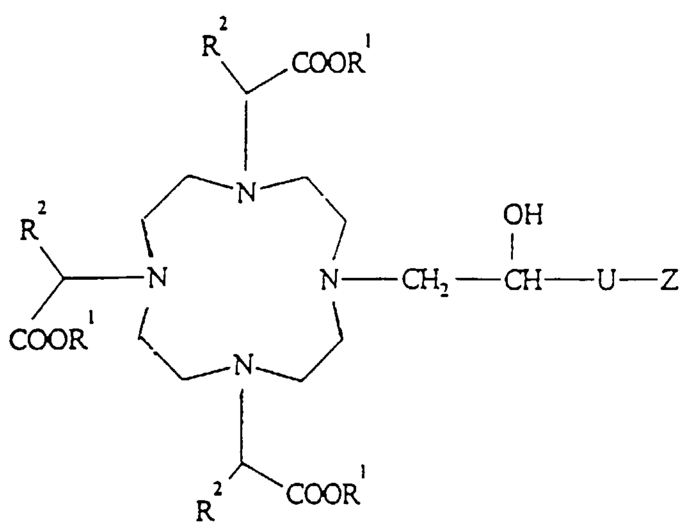

(IV)

où
$R^1$ indépendamment les uns des autres, représentent l'hydrogène ou un équivalent d'ion métallique des éléments avec le nombre atomique de 21 à 29, 39, 42 à 44 ou 57 à 83,
$R^2$ représente un atome d'hydrogène, un radical méthyle ou un radical éthyle qui est éventuellement substitué par 1 ou 2 groupes hydroxyle,
U représente un groupe alkylène en $C_1$-$C_{20}$ linéaire, ramifié, saturé ou insaturé contenant éventuellement des groupes imino, phénylène, phénylènoxy, phénylènimino, amido, hydrazido, carbonyle, ester, des atomes d'oxygène, de soufre et/ou d'azote, éventuellement substitué par des groupes hydroxy, mercapto,

oxo, thioxo, carboxy, carboxyalkyle, ester et/ou amino et

Z représente un groupe -CO-, -NH-CO- ou -NHCS-,

les complexes contenant au moins 8 à 64 ions métalliques des éléments précités et des groupes carboxyle libres, si on le souhaite, sous forme de sels d'une base organique ou inorganique ou d'aminoacides.

**2.** Complexes de polymères dendrimères selon la revendication 1, caractérisés en ce que le noyau A représente un atome d'azote, un radical groupe

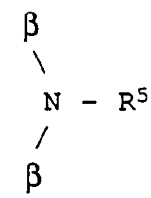

ou un groupe $\beta$-N-$(R^5)_2$. Toutefois, un radical de formule générale V, VI, VII ou VIII

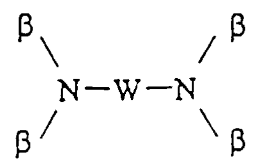

(V)

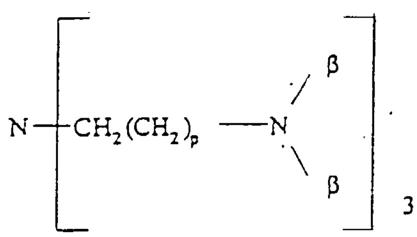

(VI)

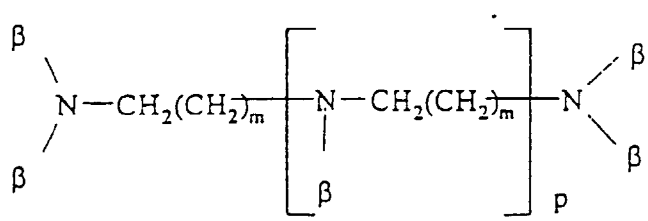

(VII)

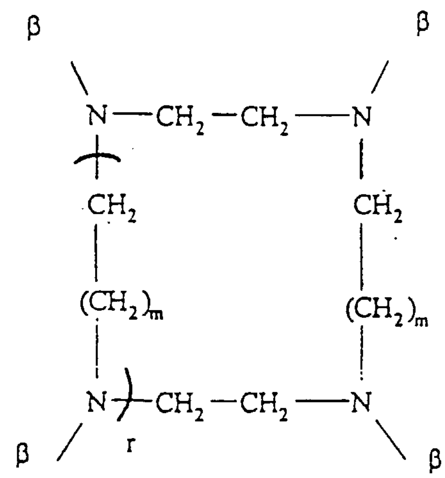

(VIII)

est particulièrement approprié, dans lesquelles

- $R^5$ représente un radical aralkyle, aryle ou alkyle avec jusqu'à 12 atomes de carbone, éventuellement substitués par 1 à 4 groupes OH,
- β désigne le site de liaison au radical X, le nombre des β devant être égal à la multiplicité de bases b,
- W représente un radical aralkylène, arylène ou alkylène avec jusqu'à 12 atomes de carbone, linéaire ou ramifié, qui est éventuellement interrompu par 1 à 4 atomes d'oxygène et/ou substitués par 1 à 4 groupes hydroxyle,
- p va de 1 à 4,
- m indépendamment les uns des autres représentent 1 ou 2 et
- r va de 1 à 5.

3. Complexes de polymères dendrimères selon les revendications 1 et 2, caractérisés en ce que le noyau A représente un groupe $>N-(CH_2)_4-N<$.

4. Produits de diagnostic contenant au moins un complexe de polymère dendrimère selon les revendications 1 à 3, dans un milieu physiologiquement tolérable, éventuellement avec des additifs usuels en galénique.

5. Utilisation d'un complexe de polymère dendrimère selon la revendication 1 pour la préparation de produits pour le diagnostic par la RMN ou aux rayons X.

6. Procédé pour la préparation d'un complexe polymère dendrimère selon les revendications 1 à 3, caractérisé en ce qu'on fait réagir le polymère dendrimère de formule IX

$$A\text{-}(X')_b \qquad \text{(IX)}$$

dans laquelle

A et b ont les significations données dans la revendication 1 et
X' représente X, cependant à la différence de X, pour la n-ième génération, les positions α ne sont pas occupées par des éléments inducteurs d'image Y mais par des atomes d'hydrogène, avec un précurseur réactif de l'agent complexant ou du complexe inducteur d'image dans une réaction d'acylation ou d'addition, et ensuite, s'il s'agit d'un agent complexant dans le cas du précurseur réactif, avec des sels métalliques ou oxydes métalliques des métaux précités pour obtenir le complexe voulu.

7. Procédé selon la revendication 6, contenant comme précurseur réactif un complexe ou produit complexant contenant un groupe activé acide carboxylique, isocyanato ou isothiocyanato.